# EUROPÄISCHE PATENTANMELDUNG

(11) **EP 1 632 219 A1**
(43) Veröffentlichungstag der Anmeldung: **08.03.2006**
(21) Anmeldenummer: 05105606.7
(22) Anmeldetag: 23.06.2005
(51) Int. Cl.: A61K 8/73, A61Q 5/06, A61K 8/81, A61K 8/04

(54) **Zubereitung zur Fixierung keratinischer Fasern mit einer Kombination aus fixierenden Polymeren und Cellulosederivaten**

(30) Priorität: 25.08.2004 DE 102004041295
(71) Anmelder: Beiersdorf AG, 20253 Hamburg (DE)
(72) Erfinder: DETERT, Marion, 22455, Hamburg (DE); SASS, Viola, 25488, Holm (DE)

(57) **Zusammenfassung**

Zubereitung zur Fixierung keratinischer Fasern umfassend
a) zwei festigende Polymere,
b) zwei Cellulosederivate,
c) ein Tensid.

## Beschreibung

Die vorliegende Erfindung betrifft Stylingmittel mit einer Kombination aus fixierenden Polymeren und Cellulosederivaten, insbesondere Schaumfestiger zur Anwendung auf dem Haar.
Eine Zubereitung zur Fixierung keratinischer Fasern im Sinne der vorliegenden Schrift kann ein Haarfestiger, auch Festigerschaum oder Schaumfestiger, aber auch eine nicht schäumende oder nicht aufgeschäumte Zubereitung sein.
Kationische Polymere im Sinne der vorliegenden Schrift sind makromolekulare Substanzen, die mehr positive als negative Ladungen im Molekül selbst, d.h. ohne Berücksichtigung von Gegenionen, aufweisen.
Die mittlere Molmasse im Sinne der vorliegenden Schrift stellt das Gewichtsmittel der Molmassen dar.
Prozent (%) im Sinne der vorliegenden Schrift sind Gewichtsprozent.
Zur temporären Verformung und Stabilisierung der Frisur werden Produkte eingesetzt, die als Stylingmittel bekannt sind. Hierzu zählen u. a. auch Haarfestiger oder Festigerschäume. Diese können entweder als aerosolhaltige Zubereitung in einer entsprechenden Verpackung vorliegen, wobei das Produkt bei der Entnahme durch das Aerosol aufschäumt oder in aerosolfreier Form in einem besonderen Packmittel vorliegen, wobei bei der Entnahme das Aufschäumen durch den besonderen Spender (Pumpfoamer oder Aufschäumspender), oder durch Versprühen einer Lösung durch eine Sprühpumpe erfolgt.
Üblicherweise bestehen diese Mittel zur Festigung der Haare aus alkoholischen oder wässrig alkoholischen Lösungen von filmbildenden natürlichen oder synthetischen Polymeren, auch als fixiernde Polymere bezeichnet. Als solche Polymere werde oft nichtionische, amphotere, anionische oder kationische Polymere eingesetzt, die für die Halteeigenschaften verantwortlich sind. Gerade bei Haarfestigern, insbesondere bei aufschäumenden oder aufgeschäumten Produkten sind die pflegenden Eigenschaften, wie z.B. gute Kämmbarkeit der Haare im nassen und trocknen Zustand, der Griff der Haare im nassen und trocknen Zustand wichtige Kriterien, die einen "gepflegten" oder "gesunden" Zustand der Haare charakterisieren.
Der Einsatz von festigenden Polymeren fixiert die erstellte Frisur, lässt das Haar aber eher belegt und rau erscheinen. Der Griff der Haare ist nicht mehr glatt und gepflegt. Die Kämmbarkeit der Haare, weder trocken, noch nass, wird nicht verbessert. Daher sind die pflegenden Eigenschaften bei herkömmlichen Haarfestigern noch nicht optimal.
Der Zusatz von konditionierenden Rohstoffen wiederum beschwert das Haar oft und verringert so das Volumen der Frisur.
EP 1340485 A2 betrifft eine Schaumzubereitung mit Treibgas und Flüssiger Phase, die ein Tensid, ein kationische Vinylpyrrolidinpolymer und eine Zelluloseverbindung enthält.

Es hat sich für den Fachmann nicht vorhersehbar herausgestellt, dass eine Zubereitung zur Fixierung keratinischer Fasern umfassend
a) zwei festigende Polymere,
b) zwei Cellulosederivate,
c) ein Tensid den Mängeln des Standes der Technik abhelfen, indem sie die negativen Eigenschaften üblicher Festiger verbessern. Das Produkt wird als Schaum auf die Haare aufgetragen. Die erfindungsgemäße Kombination führt dazu, dass der Haarfestiger neben den guten Halteeigenschaften das Haar mit einem guten Griff und einer guten Kämmbarkeit des nassen und trockenen Haares versorgt ohne es unnötigerweise zu beschweren. Das Haar wirkt nicht mehr belegt, sondern gepflegt. Ihm kommt somit eine überragende pflegende Leistung zu, wobei die Stylingeigenschaften des Produktes nicht negativ beeinträchtigt werden. Das Haar ist nicht beschwert und die Frisur erhält das gewünschte Volumen.

Die Cellulosederivate werden ausgesucht unter den nichtionischen, anionischen oder kationischen Cellulosederivaten (bevorzugt wird die Kombination aus HPMC (Methocel E 4 M Premium; Fa. Nordmann & Rassmann) und Polyquaternium-4 (Celquat -L200; Fa. National Starch). Das Produkt wird bevorzugt als schäumendes Produkt (Pumpfoamer oder Aerosol) auf das Haar aufgebracht.
Es wurde weiter gefunden, dass es bevorzugt ist, wenn als
a)zwei festigende Polymere
   a1) 0,05 ― 10%, besonders bevorzugt 0,1-5% kationisches Poymer mit einem mittleren Molmasse von 75.000 - 150.000, bevorzugt 100.000, besonders bevorzugt Luviquat FC 370; Fa. BASF und
   a2) 0,05 ― 15%, besonders bevorzugt 1-10% nichtionisches Polymer aufgebaut vollständig oder teilweise aus Vinyllactammonomeren,
b) als zwei Cellulosederivate
   b1) 0,05- 10%, besonders bevorzugt 0,1-5%, nichtionisches Cellulosederivat,
   b2) 0,05- 10%, besonders bevorzugt 0,1-5% quaternisiertes Cellulosederivat,
c) als Tensid 0,05 - 5%, besonders bevorzugt 0,1-1% amphoteres, nichtionisches, anionisches oder kationisches Tensid oder eine Kombinatione aus diesen Tensiden verwendet werden. Weiter bevorzugt ist es, wenn das kationische Polymer aus der Gruppe der Methvinylimidazoliumchlorid/Vinylpyrrolidon Copolymere, bevorzugt PQ-16 ausgewählt wird. Weiter bevorzugt ist es, wenn das nichtionische Polymer teilweise oder vollständig aus Vinyllactammonomeren, bevorzugt VP/VA Copolymer besteht. Bevorzugt ist auch, wenn als nichtionisches Cellulosederivat eine Hydroxyalkylalkylcellulose, bevorzugt Hydroxypropylmethylcellulose ausgewählt wird, beispielsweise Methocel E 4 M Premium, Fa. Nordmann & Rassmann. Darüber hinaus ist es bevorzugt, wenn als quaternisiertes Cellulosederivat Polyquaternium-4 ausgewählt wird, zum Beispiel Celquat L-200, Fa. National Starch. Bevorzugt ist auch, wenn als Tensid ein nichtionisches Tensid, besonders bevorzugt PEG-40 Hydrogenated Castor oil ausgewählt wird. Bevorzugt ist es weiterhin, wenn eine erfindungsgemäße Zubereitung zusätzlich bis 15%, besonders bevorzugt 5 bis 10% Treibmittel enthält. Die Erfundung umfasst auch eine erfindungsgemäße Zubereitung enthalten in einem Aufschäumspender sowie die Verwendung einer erfindungsgemäßen Zubereitung als Haarfestiger.

Als Copolymer aus Vinylpyrrolidon und quaternisierten Vinylimidazol im Sinne der vorliegenden Erfindung können die Produkte Luviquat FC 370 (Fa. BASF), Luviquat FC 550 (Fa. BASF), Luviquat HM 552 (Fa. BASF), Luviquat FC 905 (Fa. BASF), Luviquat Hold, Luviquat MS 370 eingesetzt werden. Besonders bevorzugt ist Luviqaut FC 370 von BASF
(INCl: Polyquaternium-16).
Als nichtionische Polymer im Sinne der vorliegenden Erfindung werden Homopolymere des Vinylpyrrolidons, wie z.B. die Luviskol K Typen von BASF oder Copolymerisate des Vinylpyrrolidons und Vinylacetat wie z.B. die Luviskol VA Typen von BASF eingesetzt. Besonders bevorzugt ist Luviskol VA 64W von BASF (INCI: VP/VA Copolymer).

Als Cellulosederivate im Sinne der vorliegenden Erfindung können Celluloseether wie Hydroxypropylcellulose, Hydroxyethyllcellulose, Hydroxymethylcellulose, Hydroxypropylmethylcellulose, Hydroxybutylcellulose oder aber quaternisierte Cellulosederivate wie z.B. Ucare Polymer JR, Fa. Union Carbide,Celquat L-200 oder Celquat H-100 (INCI: Polyquaternium-4) der Fa. National Starch. Bevorzugt eingesetzt wird eine Kombination aus Hydroxypropyl Methylcellulose wie Methocel E4M Premium von Dow Chemical und einer quaterniesierten Cellulose wie Celquat-L-200 von National Starch.

Das erfindungsgemäße Mittel enthält anionische, kationische, amphotere oder nichtionische Tenside:
Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und ― je nach Wunsch ― für Schaumregulierung.

Bei den hydrophilen Anteilen eines Tensidmoleküls handelt es sich meist um polare funktionelle Gruppen, beispielweise ―COO⁻, **―**OSO₃²⁻, ―SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2) X⁻ = beliebiges Anion, z.B. Cl⁻

RNH₂⁺CH₂CH₂COO⁻ (bei pH=7)

RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wässrigem Medium keine lonen.

### A. Anionische Tenside

Vorteilhaft zu verwendende anionische Tenside sind

Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.

sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Als nichtionische Tenside im Sinne der vorliegenden Erfindung können Fettalkoholethoxylate, Fettsäureethoxylate, Fettsäuremonoglyceride, Fettsäureglycolpartialester oder Polyoxyethylenglycolfettsäureeste eingesetzt werden. Vorteilhaft ist die Verwendung von nichtionischen Tensiden mit einem HLB Wert der größer als 12 ist, bevorzugt 13 ― 16.

Bevorzugt eingesetzt wird PEG-40 Hydrogenated Castor Oil wie z.B: Cremophor RH 410 von Fa. BASF oder Sympatens TRH/400 von Fa. Kolb.

Als Treibmittel im Sinne der vorliegenden Erfindung können Dimethylether oder Kohlenwasserstofftreibgase wie z.B. n-Butan, i-Butan, Propan sowie komprimierte Gase wie N₂, N₂ O, CO₂ eingesetzt werden.

Als Aufschäumspender im Sinne der vorliegenden Erfindung können Pumpfoamer mit dem Schaumkopf WRF3 von der Fa. Airspray eingesetzte werden.
Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.
Optionale herkömmliche Additive können ebenfalls in die Formulierung einbezogen sein, um der Zusammensetzung bestimmte Modifizierungseigenschaften zu verleihen: dies sind Silikone oder Silikonderivate, Benetzungsmittel, Feuchthaltemittel, Weichmacher wie Glycerin, Glykol und Phthalester und ― ether, erweichende Mittel, Riechstoffe und Parfüms, UV- Absorber, Farbstoffe , Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, oberflächenaktive Mittel, Viskositätsmodifizierer, Verdicker, Geliermittel, Trübungsmittel, Stabilisatoren, Sequestierungsmittel, Komplexbildner, Perlglanzmittel, ästhetische Verstärker, botanische Extrakte und Klärhilfsmittel.
Diese Additive sind im allgemeinen in einer Konzentration von etwa 0,01% bis 10 Gew.% bezogen auf das Gesamtgewicht der Zusammensetzung vorhanden

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### Beispiele 1―4: Haarfestiger

| | **1** | **2** | **3** | **4** |
|---|---|---|---|---|
| Polyquaternium-11 | 2,0 | - | - | - |
| Polyquaternium-16 | - | 1,0 | 2,0 | 1,5 |
| Polyquaternium-4 | 0,5 | 1,0 | 1,0 | 0,5 |
| VP/VA Copolymer | 3,0 | 4,0 | 5,0 | 5,0 |
| Hydroxyethylcellulose | 0,1 | 0,2 | - | - |
| Hydroxypropylmethylcellulose | - | - | 0,3 | 0,2 |
| pH Einstellung | q.s. | q.s. | q.s | q.s. |
| PEG-40 Hydrogenated Castor Oil | - | 0,2 | 0,15 | 0,15 |
| Ceteareth-20 | 0,15 | - | - | - |
| Parfüm | 0,15 | 0,15 | 0,15 | 0,15 |
| Cetrimoniumchloride | 0,1 | - | 0,1 | 0,1 |
| Dimethicone | - | 0,1 | - | - |
| Ethanol | 10,0 | - | 5,0 | - |
| Treibmittel | 10,0 | 10,0 | 10,0 | 10,0 |
| Wasser | ad 100 | ad 100 | ad 100 | ad 100 |

### Beispiele 5―8: Haarfestiger

| | 5 | 6 | 7 | 8 |
|---|---|---|---|---|
| Polyquaternium-11 | 2,0 | - | - | 0,5 |
| Polyquaternium-16 | - | 1,0 | 2,0 | 1,5 |
| Polyquaternium-4 | 0,5 | 1,0 | 1,0 | 1,0 |
| VP/VA Copolymer | - | 4,0 | 5,0 | 5,5 |
| Hydroxypropylmethylcellulose | 0,5 | 0,5 | 0,4 | 0,2 |
| pH Einstellung | q.s. | q.s. | q.s. | q.s. |
| PVP | 3,0 | - | - | - |
| PEG-40 Hydrogenated Castor Oil | - | 0,15 | 0,15 | 0,1 |
| Ceteareth-20 | 0,15 | - | - | - |
| Parfüm | 0,15 | 0,15 | 0,15 | 0,1 |
| Cetrimoniumchloride | 0,1 | 0,1 | 0,1 | 0,15 |
| Dimethicone | - | - | - | 0,1 |
| Ethanol | 10,0 | - | 5,0 | - |
| Treibmittel | - | - | - | 10,0 |
| Wasser | ad 100 | ad 100 | ad 100 | ad 100 |

## Patentansprüche

1. Zubereitung zur Fixierung keratinischer Fasern umfassend
a) zwei festigende Polymere,
b) zwei Cellulosederivate,
c) ein Tensid.

2. Zubereitung nach Anspruch 1 **dadurch gekennzeichnet, dass**
a) als zwei festigende Polymere
a1) 0,05 ― 10% kationisches Poymer mit einem mittleren Molmasse von 75.000 - 150.000,
a2) 0,05 ― 15% nichtionisches Polymer aufgebaut vollständig oder teilweise aus Vinyllactammonomeren,
b) als zwei Cellulosederivate
b1) 0,05 ― 10% nichtionisches Cellulosederivat,
b2) 0,05 ― 10% quaternisiertes Cellulosederivat,
c) als Tensid 0,05 ― 5% amphoteres, nichtionisches, anionisches oder kationisches Tensid oder eine Kombinatione aus diesen Tensiden verwendet werden.

3. Zubereitung nach Anspruch 2 **dadurch gekennzeichnet, dass** das kationische Polymer aus der Gruppe der Methvinylimidazoliumchlorid/Vinylpyrrolidon Copolymere, bevorzugt PQ-16 ausgewählt wird.

4. Zubereitung nach Anspruch 2 oder 3 **dadurch gekennzeichnet, dass** das nichtionische Polymer teilweise oder vollständig aus Vinyllactammonomeren, bevorzugt VP/VA Copolymer besteht.

5. Zubereitung nach Anspruch 2,3 oder 4 **dadurch gekennzeichnet, dass** als nichtionisches Cellulosederivat eine Hydroxyalkylalkylcellulose, bevorzugt Hydroxypropylmethylcellulose ausgewählt wird.

6. Zubereitung nach Anspruch 2,3,4,5 oder 6 **dadurch gekennzeichnet, dass** als quaternisiertes Cellulosederivat Polyquaternium-4 ausgewählt wird.

7. Zubereitung nach Anspruch 2,3,4,5 oder 6 **dadurch gekennzeichnet, dass** als Tensid ein nichtionisches Tensid, bevorzugt PEG-40 Hydrogenated Castor oil ausgewählt wird ist.

8. Zubereitung nach einem der vorangehenden Ansprüche **dadurch gekennzeichnet, dass** zusätzlich bis 15% Treibmittel enthalten sind.

9. Zubereitung nach einem der vorangehenden Ansprüche enthalten in einem Aufschäumspender.

10. Verwendung einer Zubereitung nach einem der vorangehenden Ansprüche als Haarfestiger.
